# EUROPEAN PATENT SPECIFICATION

(11) **EP 1 475 046 B1**
(45) Date of publication and mention of the grant of the patent: **23.09.2009**
(21) Application number: 04010612.2
(22) Date of filing: 05.05.2004
(51) Int. Cl.: A61B 17/06

(54) **Suture material dispenser**
Nahtmaterialverpackung
Emballage de suture

(30) Priority: 08.05.2003 DE 10320463
(43) Date of publication of application: 10.11.2004
(73) Proprietor: B. Braun Surgical, S.A., 08191 Rubi (Barcelona) (ES)
(72) Inventor: Aranda Garcia, Jose Antonio, Terrassa 08202 (ES); Afonso Sanmarti, Olga, Sabadell 08226 (ES)
(74) Representative: Patentanwälte Ruff, Wilhelm, Beier, Dauster & Partner

(56) References cited:
- EP-A- 0 529 297
- US-A- 5 236 083
- US-A- 5 655 652

## Description

The present invention relates to a suture material dispenser for surgical suture material, comprising a winding body having a central region with a winding channel extending therearound for receiving the suture material, the winding channel being defined by two side walls.

Described in EP 0 914 802 A2 (B. Braun Surgical, S.A.) is a suture material dispenser comprising two component plates of flexible hard shell which are provided with mutually engaging deformations and thus are connected to each other. The plates are formed with edge strips diverging towards the outside and forming a winding channel. At the bottom of the winding channel, a flexible gap is provided, allowing the suture material to enter a continuously surrounding accommodation area forming a closed space.

Document US-A-5 236 083, on whose disclosure the preamble of claim 1 is based, describes a one piece needle and suture dispenser in which a rounded channel is formed for retention of the suture.

It is an object of the instant invention to provide a suture material dispenser in which the suture material, comprising one or a plurality of threads, can be inserted into the winding body in a simple manner and be safely stored within a closed winding channel.

The suture material dispenser according to the instant invention is defined by the features of claim 1. Thus, at least one of the side walls of the winding channel is arranged to be pivoted relative the other side wall so that the winding channel can assume an opened position in which the outer end regions of the side walls are spaced from each other, and a closed position in which the outer end regions of the side walls abut each other.

The suture material dispenser of the invention makes it possible to wind the suture material into the winding channel by means of a winding machine while the side walls of the winding channels are in their open position. Upon termination of the winding process, the side walls are moved into the closed position so that the winding channel with form a continuously surrounding hollow space of substantially closed cross section. The suture material cannot inadvertently slide out of this hollow space. Only by pulling the thread end provided for this purpose, suture material can be dispensed.

With the present invention, the wind-up process can be performed in a simple manner. The thread material will be simply wound into the opened winding channel of the winding body. Subsequently, the side walls of the winding channel will be pressed against each other to form a closed channel safely enclosing the suture material.

According to a preferred embodiment of the invention, the winding body comprises a one-pieced plastic member, with the pivotable side wall extending therefrom via a living hinge. The living hinge acts as a flexible joint allowing the respective side wall of the winding channel to be pivoted. The living hinge comprises a weakening line in the one-pieced plastic member. Preferably, this weakening line is realized by a continuously surrounding groove.

According to a further embodiment, the invention relates to a suture material dispenser characterized by the distinctive feature that the side walls of the winding channel are provided with openings for the passage of winding pins of a winding machine.

The suture material dispenser will be inserted into the winding machine, with the winding pins of the winding machine forming the winding core. After the thread material has been wound up, the suture material dispenser is removed from the winding pins while the suture material is stripped off the winding pins and then remains in the dispenser.

Preferably, the winding channel has a bottom which in regions between two openings is formed to present raised portions configured to deflect the suture material. The raised portions between two winding pins will effect a polygonal course of the thread coil. Thereby, it is accomplished that the thread coil will abut the winding body and the winding pins only in places and can be easily lifted off the winding pins without interference due to friction of the suture material.

The central region of the winding body can be provided with a needle holder formed as a one-pieced unit with the winding body. It is of advantage that the needle holder is formed integrally with the winding body during the molding of the latter and that no separate mounting process is needed for the needle holder. Further, it is safeguarded that the needle holder is undetachably and firmly connected to the winding body.

The needle holder preferably comprises a cantilevered arm which in combination with a support face forms a tapering clamping gap. The clamping gap serves for clamping fixation of a needle or a thread.

A preferred embodiment will be explained in greater detail hereunder with reference to the drawings. The particular features of the exemplary embodiment are not to be construed as delimiting the protective scope of the patent. The scope of protection is defined by the claims.
- Fig. 1: is a perspective view of the suture material dispenser,
- Fig. 2: is a bottom view of the suture material dispenser,
- Fig. 3: is a sectional view of the suture material dispenser taken along the line III-III of Fig. 1,
- Fig. 4: is a sectional view taken along the line IV-IV of Fig. 3,
- Fig. 5: is a sectional view taken along the line V-V of Fig. 3,
- Fig. 6: is a sectional view of the winding channel in the closed position similar to Fig. 4, and
- Fig. 7: is a sectional view of the winding channel in the closed position similar to Fig. 5.

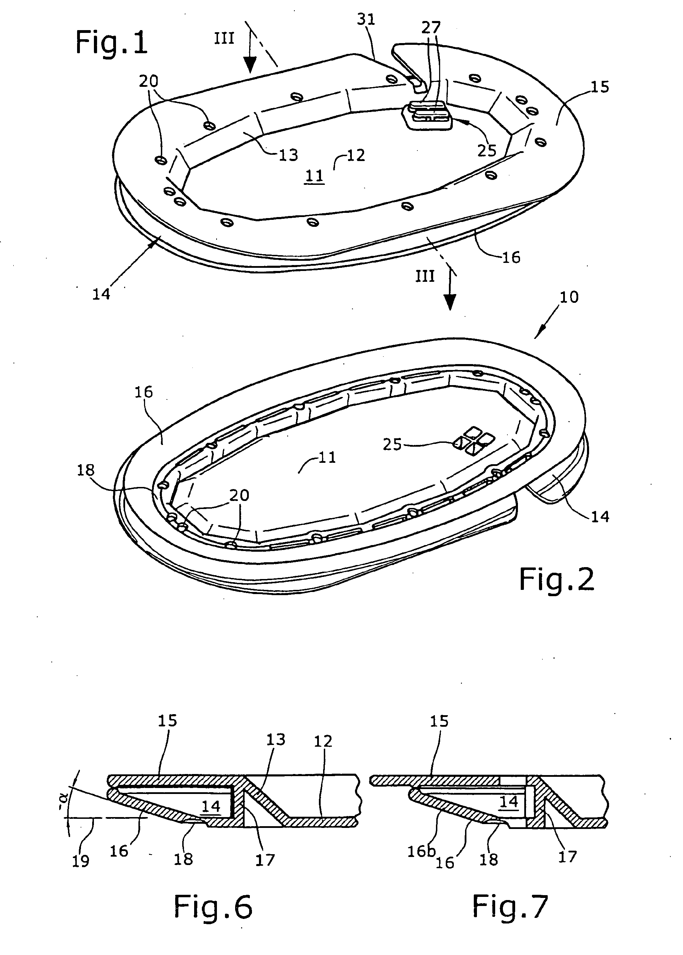

The suture material dispenser comprises a winding body 10 formed as a one-pieced plastic member in an injection molding process. The winding body 10, of which the top side is shown in Fig. 1 and the bottom side in Fig. 2, has a central region 11 comprising a plane plate 12 and a slope 13 obliquely rising from the edge of plate 12.

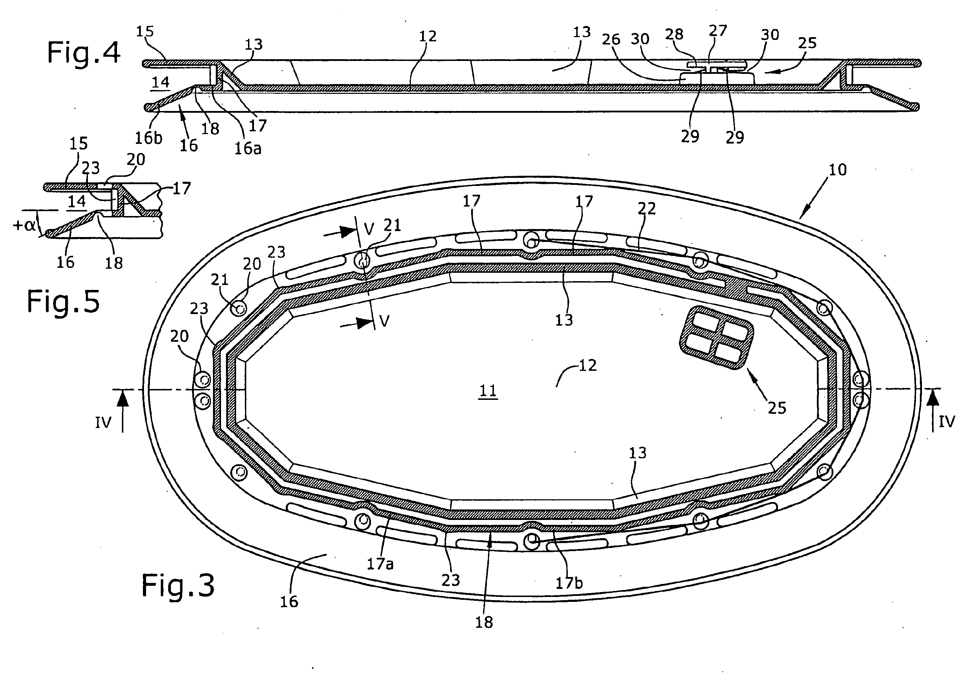

The outer periphery of the slope 13 merges with the winding channel 14. The winding channel 14 is delimited by two side walls 15,16 and a bottom wall 17 connecting the side walls to each other. As illustrated in Figs. 4 and 5, winding channel 14 in its open position has a U-shaped cross section, with its opening oriented to face radially outwards.

In plan view, the whole winding body 10 has an oval shape, while also the central region 11 has an oval shape and the bottom 17 of winding channel 14 is arranged along a substantially oval course. Alternatively, also a circular shape of the winding body can be provided.

As evident from Figs. 4 and 5, the side wall 15 of winding channel 14 extends generally in parallel to the plate 12 of central region 11 but at a displacement in height. The other side wall 16 comprises a first portion 16a adjoining the bottom 17 and arranged in the plane of plate 12, and a portion 16b extending obliquely outwards from first portion 16a. Portion 16b is formed with an outward divergence from side wall 15, thus creating a funnel-shaped opening of winding channel 14. Formed between portions 16a and 16b on their underside is a groove 18 acting as a living hinge for pivoting the portion 16b therearound. As seen in Fig. 2, groove 18 runs along the complete circumference of winding body 10. Groove 18 allows the portion 16b to be folded from the opened position shown in Figs. 4 and 5 into the closed position shown in Figs. 6 and 7. Each of these positions is provided as a stable orientation in which the side wall 16 will remain, unless subjected to external forces.

In the open position of winding channel 14 according to Figs. 4 and 5, the suture material can be wound into the winding channel. Then, by pivoting the portion 16b of side wall 16, the winding channel 14 will be closed as illustrated in Figs. 6 and 7.

The pivotable side wall 16 continuously surrounds the complete circumference, i. e. it does not contain any gaps. Side wall 16 displays a bistable behaviour. It is moved between the open position shown in Fig. 5 and the closed position shown in Figs. 6 and 7, which merely requires said wall to be folded once. The wall then snaps, via an instable central position, from the open position into the closed position. In the open position shown in Fig. 5 the wall 16 has an angle of +α relative to a plane 19 which extends in parallel to the plane of the plate 12 of the central region. In the closed position shown in Fig. 6 the wall 16 assumes an angle of -α relative to the plane 19. The amounts of the two angles need not be the same. It is of importance that the two angles have different signs such that the snapping effect of the bistability is attained. Thus only the open position and the closed position of the wall 16 are stable.

Winding channel 14 has a plurality of transverse openings 20 extending therethrough which are arranged in the vicinity of bottom 17 and formed to pass through both side walls 15 and 16. The groove 18 provided on the underside of portions 16a,16b traverses these openings 20 by a part of its cross-section, as seen in Fig. 5.

The openings 20 are provided to have winding pins 21 of a winding machine inserted therethrough (Fig. 3) so that the thread-shaped suture material 22 can be wound about the winding pins.

As illustrated in Fig. 3, the bottom 17 of winding channel 14 is formed to take a polygonal course around central region 11. Thus, between linear stretches 17a, raised portions 23 are provided in the shape of edgy protrusions deflecting the course of the suture material 22. As a result, the suture material 22 will abut the winding pins 21 and raised portions 23 only in places. Each winding pin 21 is arranged substantially in the central region of a linear stretch, i.e. between two raised portions 23.

Provided on the upper side of plate 12 is a suture material holder 25, forming an integral component of winding body 10. Suture material holder 25 comprises a base portion 26 (Fig. 4) with two T-shaped structures 27 projecting therefrom. Each of the structures 27 carries two cantilevered arms 28 extending to opposite sides. Between these structures, an obliquely ascending support face 29 is provided. Together with the two adjacent cantilevered arms 28, support face 29 forms a clamping gap 30 for clamping fixation of suture material, i.e. a thread or a needle.

Near the suture material holder 25, the upper side wall 15 of winding channel 14 is formed with a recess 31 through which the suture material can be guided out of winding channel 14 for fixing the end of the suture material on the suture material holder 25.

For removing suture material from the dispenser, the end of the suture material fixed in the suture material holder 25 is disengaged, and the thread is pulled by this end to withdraw a length of thread from the winding channel 14.

The suture material dispenser is of a flat and place-saving configuration. Production of the dispenser is possible in a simple manner and at low cost. The suture material accommodated in the dispenser will be safely protected therein. After the suture material has been wound up, the dispenser can be taken out of the winding machine easily and without substantial hindrances.

## Claims

1. A suture material dispenser, comprising a winding body (10) with a central region (11) and with a winding channel (14) surrounding the central region (11) for receiving the suture material (22), the winding channel (14) being defined by two side walls (15,16),
wherein
at least one of the side wails (16) is arranged to be pivoted relative to the other side wall (15) for moving the winding channel (14) Into an opened potion in which the outer end regions of the side walls (15,16) are spaced from each other, and a closed position in which the outer end regions of the side walls (15,16) abut each other, **characterised in that** the at least one side wall (16) displays a bistable behaviour and can be snaped via an instable central position from the open position into the closed position.

2. The suture material dispenser according to claim 1, wherein the winding body (10) comprises a one-pieced plastic member and the pivotable side wall (16) extends from a living hinge (18).

3. The suture material dispenser according to claim 1 or 2, wherein the pivotable side wall (16) is a continously surrounding wall.

4. The suture material dispenser according to one of claims 1-3, wherein the pivotable side wall (16) with the bistable behaviour assumes in both positions angles (+α,-α) with opposite signs relative to a plane (19) which extends in parallel to the plane of the central region (11).

5. The suture material dispenser according to claim 2, wherein the living hinge is formed by a surrounding groove (18).

6. The suture material dispenser, according to one of claims 1 to 5
wherein the side walls (15,16) of the winding channel (14) are formed with openings (20) for the passage of winding pins (21) of a winding machine.

7. The suture material dispenser according to claim 6, wherein the winding channel (14) has a bottom (17) which In regions between two openings (20) forms a raised portion (23) deflecting the suture material (22).

8. The suture material dispenser according to any one of claims 1-7, wherein the central region (11) comprises a plane plate (12) and a slope (13) obliquely rising from the plate (12) and merging with the bottom (17) of the winding channel (14).

9. The suture material dispenser according to any one of claims 1-8, wherein the central region (11) comprises a suture material holder (25) integrally formed thereto.

10. The suture material dispenser according to claim 7,
wherein the suture material holder (25) comprises a cantilevered arm (28) which together with a support face (29) forms a tapering clamping gap (30).

## Patentansprüche

1. Nahtmaterialspender, der einen Wickelkörper (10) mit einer zentralen Region (11) und einem Wickelkanal (14), der die zentrale Region (11) zur Aufnahme von Nahtmaterial (22) umgibt, umfasst, wobei der Wickelkanal (14) von zwei Seitenwänden (15, 16) definiert wird, worin zumindest eine der Seitenwände (16) relativ zur anderen Seitenwand (15) geschwenkt werden kann, um den Wickelkanal (14) in eine offene Stellung, in der die äußeren Endregionen der Seitenwände (15, 16) im Abstand zueinander liegen, und in eine geschlossene Stellung, in der die äußeren Endregionen der Seitenwände (15, 16) aneinander stoßen, zu bewegen, **dadurch gekennzeichnet, dass** die zumindest eine Seitenwand (16) ein bistabiles Verhalten zeigt und über eine instabile zentrale Stellung von der offenen Stellung in die geschlossene Stellung gebracht werden kann.

2. Nahtmaterialspender nach Anspruch 1, worin der Wickelkörper (10) ein einteiliges Plastikelement umfasst und sich die schwenkbare Seitenwand (16) von einem Filmscharnier (18) aus erstreckt.

3. Nahtmaterialspender nach Anspruch 1 oder 2, worin die schwenkbare Seitenwand (16) eine kontinuierliche Umgebungswand ist.

4. Nahtmaterialspender nach einem der Ansprüche 1-3, worin die schwenkbare Seitenwand (16) mit bistabilem Verhalten in beiden Stellungen Winkel (+α, -α**)** mit entgegengesetzten Vorzeichen relativ zu einer Ebene (19), die parallel zur Ebene der zentralen Region (11) verläuft, annimmt.

5. Nahtmaterialspender nach Anspruch 2, worin das Filmscharnier von einer Umgebungskerbe (18) geformt wird.

6. Nahtmaterialspender nach einem der Ansprüche 1 bis 5, worin die Seitenwände (15, 16) des Wickelkanals (14) mit Öffnungen (20) zur Durchführung von Wickelstiften (21) einer Wickelmaschine geformt sind.

7. Nahtmaterialspender nach Anspruch 6, worin der Wickelkanal (14) einen Boden (17) aufweist, der in Regionen zwischen zwei Öffnungen (20) einen erhabenen Abschnitt (23) bildet, der das Nahtmaterial (22) umlenkt.

8. Nahtmaterialspender nach einem der Ansprüche 1-7, worin die zentrale Region (11) eine ebene Platte (12) und eine Schräge (13) umfasst, die sich schräg von der Platte (12) aus erhebt und in den Boden (17) des Wickelkanals (14) übergeht.

9. Nahtmaterialspender nach einem der Ansprüche 1-8, worin die zentrale Region (11) einen Nahtmaterialhalter (25) umfasst, der einstückig daran geformt ist.

10. Nahtmaterialspender nach Anspruch 7, worin der Nahtmaterialhalter (25) einen ausladenden Arm (28) umfasst, der zusammen mit einer Stützfläche (29) eine sich verjüngende Klemmlücke (30) bildet.

## Revendications

1. Distributeur de matière de suture, comprenant un corps de bobinage (10) présentant une région centrale (11) et possédant un canal de bobinage (14) qui entoure la région centrale (11) pour recevoir la matière de suture (22), le canal de bobinage (14) étant défini par deux parois latérales (15, 16), dans lequel:
au moins une des parois latérales (16) est agencée de manière à pouvoir pivoter par rapport à l'autre paroi latérale (15) afin de déplacer le canal de bobinage (14) dans une position ouverte dans laquelle les régions d'extrémité extérieures des parois latérales (15, 16) sont espacées les unes des autres, et une position fermée dans laquelle les régions d'extrémité extérieures des parois latérales (15, 16) butent les unes contre les autres,
**caractérisé en ce que** ladite au moins une paroi latérale (16) affiche un comportement bistable et peut être basculée de la position ouverte à la position fermée en passant par une position centrale instable.

2. Distributeur de matière de suture selon la revendication 1, dans lequel le corps de bobinage (10) comprend un élément en plastique d'une seule pièce, et la paroi latérale pivotante (16) s'étend à partir d'une charnière mobile (18).

3. Distributeur de matière de suture selon la revendication 1 ou 2, dans lequel la paroi latérale pivotante (16) est une paroi périphérique continue.

4. Distributeur de matière de suture selon l'une des revendications 1 à 3, dans lequel la paroi latérale pivotante (16) au comportement bistable adopte dans les deux positions des angles (+α, -α) ayant des signes opposés par rapport à un plan (19) qui s'étend parallèlement au plan de la région centrale (11).

5. Distributeur de matière de suture selon la revendication 2, dans lequel la charnière mobile est constituée par une rainure périphérique (18).

6. Distributeur de matière de suture selon l'une des revendications 1 à 5, dans lequel les parois latérales (15, 16) du canal de bobinage (14) comportent des ouvertures (20) pour le passage de broches de bobinage (21) d'une bobineuse.

7. Distributeur de matière de suture selon la revendication 6, dans lequel le canal de bobinage (14) présente un fond (17) qui, dans les régions situées entre deux ouvertures (20), forme une partie saillante (23) qui dévie la matière de suture (22).

8. Distributeur de matière de suture selon l'une quelconque des revendications 1 à 7, dans lequel la région centrale (11) comprend une plaque plane (12) et une pente (13) qui s'élève en oblique à partir de la plaque (12) et qui fusionne avec le fond (17) du canal de bobinage (14).

9. Distributeur de matière de suture selon l'une quelconque des revendications 1 à 8, dans lequel la région centrale (11) comprend un porte-matière de suture (25) qui est formé intégralement avec celle-ci.

10. Distributeur de matière de suture selon la revendication 7, dans lequel le porte-matière de suture (25) comprend un bras en porte-à-faux (28) qui, de concert avec une face de support (29), forme un espace de serrage conique (30).
